# EUROPÄISCHE PATENTSCHRIFT

(11) **EP 4 374 835 B1**
(45) Veröffentlichungstag und Bekanntmachung des Hinweises auf die Patenterteilung: **02.04.2025**
(21) Anmeldenummer: 24170518.5
(22) Anmeldetag: 24.06.2021
(51) Int. Cl.: A61F 9/008, A61F 9/009

(54) **VORRICHTUNG ZUM BEARBEITEN VON AUGENGEWEBE MITTELS EINES GEPULSTEN LASERSTRAHLS**
DEVICE FOR TREATING EYE TISSUE USING A PULSED LASER BEAM
DISPOSITIF DE TRAITEMENT DE TISSU OCULAIRE À L'AIDE D'UN FAISCEAU LASER PULSÉ

(30) Priorität: 25.06.2020 CH 7702020
(43) Veröffentlichungstag der Anmeldung: 29.05.2024
(62) Teilanmeldung aus: 21181555.0
(73) Patentinhaber: Ziemer Ophthalmic Systems AG, 2562 Port (CH)
(72) Erfinder: STEINLECHNER, Michael, 8006 Zürich (CH); RATHJEN, Christian, 28197 Bremen (DE)
(74) Vertreter: Rentsch Partner AG

(56) Entgegenhaltungen:
- EP-A1- 3 427 705
- US-A1- 2018 028 355

## Beschreibung

### Technisches Gebiet

Die vorliegende Erfindung betrifft eine ophthalmologische Vorrichtung zum Bearbeiten von Augengewebe mittels eines gepulsten Laserstrahls. Die vorliegende Erfindung betrifft insbesondere eine ophthalmologische Vorrichtung mit einer Laserquelle zur Erzeugung eines gepulsten Laserstrahls, einer Fokussieroptik zur Fokussierung des gepulsten Laserstrahls ins Augengewebe und ein Scannersystem, um den gepulsten Laserstrahl auf Bearbeitungspunkte auf einer Bearbeitungsbahn zu lenken.

### Stand der Technik

Zum Bearbeiten von Augengewebe mittels eines Laserstrahls wird ein Bearbeitungsbereich mit fokussiertem Laserstrahl gescannt (abgetastet), indem der in der Regel gepulste Laserstrahl mittels geeigneter Scannersysteme (Ablenkvorrichtungen) in ein oder zwei Scannrichtungen (Abtastrichtungen) abgelenkt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind, beispielsweise mit Galvanoscannern, Piezoscannern, Polygonscannern oder Resonanzscannern.

Zum Erzeugen von Schnittflächen werden durch einzelne Laserpulse getrennte Gewebebereiche so dicht nebeneinander platziert, dass ein durchgehend getrennter Gewebebereich entsteht. Zur Erzeugung von gekrümmten oder beliebig orientierten Schnittflächen ist es weiterhin erforderlich den Fokus des Laserstrahls in Strahlrichtung über divergenzmodulierende Mittel (z.B. bewegliche Linsen) oder Fokussieroptiken mit Bewegungstreibern zu verstellen. Die WO2005/048895 beschreibt beispielsweise ein System mit Spiegelablenksystem und Fokussiervorrichtung.

Als Alternative zum Scannen mit Galvanoscannern wird in der US 7,621,637 eine Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels einer mechanisch bewegten Projektionsoptik einen Arbeitsbereich gemäss einem Scannmuster abfährt. Die im Vergleich zur mechanischen Bewegung viel schnellere Ablenkung in der Scannrichtung wird im Applikationskopf auf die mechanische Bewegung der Projektionsoptik und somit auf dessen Scannmuster überlagert. Ein Fast-Scannersystem in der Basisstation ermöglicht eine feine Bewegung der Laserpulse (Microscann), welche auf das Scannmuster der beweglichen Projektionsoptik überlagert wird, die einen grossen Bearbeitungsbereich abdeckt, beispielsweise das gesamte Auge.

US 2016/0089270 beschreibt ein System und Verfahren zum Schneiden von Lentikeln im Augengewebe. Gemäss US 2016/0089270 werden dazu gradlinige Fastscannlinien langsameren Bearbeitungslinien überlagert, die entlang Meridianen des Lentikels abgefahren werden. Durch die Gradlinigkeit der Fastscannlinien entstehen Schnitte, die in ihrer Form von der gewünschten Oberflächenkrümmung des Lentikels abweichen und daher Fehler verursachen. Überdies ist zum Abfahren der Bearbeitungslinien entlang der Meridiane jeweils über die Distanz einer Lentikelbreite eine vertikale Fokusverschiebung in der Grössenordnung und im Ausmass der Dicke des zu schneidenden Lentikels erforderlich, was einerseits mit entsprechendem Aufwand und Kosten für dazu eingerichtete verschiebbare Optiken und bewegliche Linsen und andererseits mit damit einhergehenden Einbussen in der Bearbeitungsgeschwindigkeit verbunden ist. Darüber hinaus erlauben die Fastscannlinien aufgrund ihrer festen horizontalen Ausrichtung keine bestmögliche Anpassung von Schnitten an Lentikeloberflächen, insbesondere nicht, wenn diese von einer sphärischen Form abweichen.

EP3427705 beschreibt eine ophthalmologische Vorrichtung zum Bearbeiten von Augengewebe mittels eines gepulsten Laserstrahls, welche ein Scannersystem umfasst, das eingerichtet ist, den gepulsten Laserstrahl auf Bearbeitungszielpunkte entlang einer um einen Ausrichtungswinkel quer zu einer Bearbeitungslinie verlaufenden Fastscannlinie zu lenken und die Fastscannlinie abhängig vom Bearbeitungszielpunkt auf der Bearbeitungslinie derart zu verkippen, dass die Fastscannlinie im Wesentlichen entlang einer Aussenfläche eines im Augengewebe zu schneidenden Lentikels verläuft.

Derartige bekannte Systeme ermöglichen zwar die Bearbeitung von einfachen Scannmustern, beispielsweise das Schneiden eines Gewebelappens (Flap), was in der Regel als grosses Flächenstück mit einfacher Randgeometrie ausgeführt wird. Bei Anwendungen, in denen nicht nur Gewebeschnitte in einer im Wesentlichen horizontal ausgerichteten Bearbeitungsfläche auf einer gemeinsamen Fokalfläche ausgeführt werden, sondern in denen auch Schnitte mit einer vertikalen Schnittkomponente über unterschiedliche Fokushöhen ausgeführt werden sollen, z.B. schräg zur Horizontalen verlaufende oder vertikale Schnitte, erweist sich das vertikale Verfahren der Projektionsoptik oder eines Zoom-Systems für eine vertikale Veränderung des Fokus und damit der Schnitthöhe als zu langsam, um Schnitte mit einer vertikalen Komponente, also mit veränderlicher Fokustiefe während des Schneidens, mit einer Geschwindigkeit auszuführen, die vergleichbar ist mit Schnittgeschwindigkeiten in der horizontalen Bearbeitungsfläche.

Zur Erzielung kurzer Bearbeitungszeiten werden bei gegebener Schnittgeometrie Schnittführungen gesucht, die die erforderliche Verfahrgeschwindigkeiten in vertikaler Richtung minimal halten. Beispielsweise wird das Schneiden einer Kugelkalotte über eine Spirale mit monoton ansteigender bzw. abfallender Fokustiefe ohne alternierende Verfahrbewegungen realisiert.

DE102006053117 beschreibt eine Vorrichtung zur operativen Fehlsichtigkeitskorrektur eines Patientenauges, welche mittels gepulster Laserstrahlung Hornhaut-Gewebe trennt. Dabei wird die Laserstrahlung auf Zielpunkte fokussiert, die auf einem Muster in der Hornhaut liegen. Die fokussierte Laserstrahlung wird entlang einer Bahn über das Muster der Zielpunkte geführt und Laserpulse werden in der Hornhaut auch auf Punkte abgegeben, die auf der Bahn zwischen den Zielpunkten liegen.

Bei den oben angeführten ophthalmologischen Vorrichtungen zum Bearbeiten von Augengewebe mittels gepulster Laserstrahlen werden die zu bearbeitenden Augen bezüglich der ophthalmologischen Vorrichtungen fixiert. In der Regel wird dazu ein Patienteninterface verwendet, welches mittels Unterdruck am Auge fixiert wird. Für die Bearbeitung des Augengewebes ist das Patienteninterface fest oder entfernbar mit der ophthalmologischen Vorrichtung verbunden. Durch die Fixierung des zu bearbeitenden Auges bezüglich der ophthalmologischen Vorrichtung wird ermöglicht, dass das Scannersystem eine im Augengewebe zu schneidende Schnittfläche durch Ausführen eines definierten (programmierten) Schnittmusters erzeugen kann, ohne dabei durch Augenbewegungen beeinträchtigt zu werden. Problematisch erweisen sich hierbei jedoch Verkippungen des Auges bezüglich des Patienteninterfaces, beispielsweise bei einer verkippten Applikation des Patieninterfaces am Auge oder umgekehrt, da dadurch gemäss dem definierten Schnittmuster ausgeführte Schnittflächen im Augengewebe nicht an der vorgesehenen Stelle geschnitten werden.

Beispielsweise muss bei einem gekrümmten Patienteninterface dem ursprünglich definierten Scannmuster ein neues Fokustiefenprofil aufgeprägt werden. Im Falle des Schneidens einer Kugelkalotte entlang einer Spirale ergeben sich Fokusverstellbewegungen, die neben einer monoton ansteigenden bzw. abfallenden Komponente eine weitere alternierende Komponente haben. Diese alternierende Komponente alterniert mit der Umlauffrequenz der Spirale und führt zu Änderungen der Fokusverstellgeschwindigkeit während eines Umlaufs, wobei die Amplitude der Verstellung vom Grad der Verkippung abhängt. Fokusverstellantriebe können diese Geschwindigkeitsänderung nur bis zu den ihnen innewohnenden Leistungsgrenzen durchführen. Gehen die Anforderungen darüber hinaus, muss die Ausführungsgeschwindigkeit des Gesamtsystems soweit reduziert werden, bis die Fokusverstellantriebe dem Fokustiefenprofil wieder folgen können. Eine damit einhergehende Verlangsamung der Bearbeitung und Erhöhung der Behandlungszeit ist aus verschiedenen Gründen oft unerwünscht.

### Darstellung der Erfindung

Es ist eine Aufgabe der vorliegenden Erfindung eine ophthalmologische Vorrichtung zum Bearbeiten von Augengewebe mittels eines gepulsten Laserstrahls vorzuschlagen, welche zumindest einige Nachteile des Stands der Technik nicht aufweist. Es ist insbesondere eine Aufgabe der vorliegenden Erfindung eine ophthalmologische Vorrichtung zum Bearbeiten von Augengewebe mittels eines gepulsten Laserstrahls vorzuschlagen, welche ermöglicht Schnittflächen auch bei einer Verkippung des Auges bezüglich des Patienteninterfaces an der vorgesehenen Stelle im Augengewebe zu schneiden.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zum Bearbeiten von Augengewebe eines Auges, umfasst eine Laserquelle, die eingerichtet ist, einen gepulsten Laserstrahl zu erzeugen, und einen Applikationskopf mit einer Fokussieroptik und einem Patienteninterface. Die Fokussieroptik weist eine Projektionsachse auf und ist eingerichtet, den gepulsten Laserstrahl im Augengewebe auf einen Bearbeitungspunkt zu fokussieren. Das Patienteninterface weist eine Zentrumsachse auf und ist eingerichtet, den Applikationskopf am Auge zu fixieren. Die ophthalmologische Vorrichtung umfasst weiter ein Scannersystem, das eingerichtet ist, den gepulsten Laserstrahl im Augengewebe auf Bearbeitungspunkte auf einer Bearbeitungsbahn zu lenken. Die ophthalmologische Vorrichtung umfasst überdies einen Schaltkreis, der eingerichtet ist, das Scannersystem zum Schneiden einer zur Zentrumsachse des Patienteninterfaces symmetrischen Schnittfläche im Augengewebe zu steuern, wobei der gepulste Laserstrahl auf Bearbeitungspunkte auf der Schnittfläche auf einer ersten Bearbeitungsbahn gelenkt wird, und wobei die erste Bearbeitungsbahn um die Projektionsachse der Fokussieroptik herumverlaufend gekrümmt ist.

Die oben genannten Ziele werden dadurch erreicht, dass der Schaltkreis überdies eingerichtet ist, bei einer Verkippung des Auges zur Zentrumsachse des Patienteninterfaces, einen Scheitelpunkt einer verkippten Schnittfläche zu ermitteln, welche durch eine der Verkippung des Auges entsprechende Mitverkippung der Schnittfläche bestimmt ist, eine transformierte zweite gekrümmte Bearbeitungsbahn zu bestimmen, die um den Scheitelpunkt der verkippten Schnittfläche herum verläuft und Bearbeitungspunkte auf der verkippten Schnittfläche bestimmt, und das Scannersystem derart zu steuern, dass der gepulste Laserstrahl auf Bearbeitungspunkte auf der transformierten zweiten gekrümmten Bearbeitungsbahn gelenkt wird.

In einer Ausführungsvariante ist die Fokussieroptik eingerichtet, eine Bearbeitungshöhe der Bearbeitungspunkte in Richtung der Projektionsachse mit einer Fokusverstellgeschwindigkeit zu verstellen, das Scannersystem ist eingerichtet, Bearbeitungspunkte mit einer bezüglich der Fokusverstellgeschwindigkeit höheren Scanngeschwindigkeit auf der Bearbeitungsbahn zu verschieben, und der Schaltkreis ist eingerichtet, die transformierte zweite gekrümmte Bearbeitungsbahn mit Höhenänderungen in Richtung der Projektionsachse zu bestimmen, welche bei einer Bewegung von Bearbeitungspunkten mit der Scanngeschwindigkeit ohne Überschreitung der Fokusverstellgeschwindigkeit durch die Fokussieroptik verstellbar sind.

In einer Ausführungsvariante weist die erste Bearbeitungsbahn in Richtung der Projektionsachse eine stetige Höhenänderungskomponente auf, und der Schaltkreis ist eingerichtet, die transformierte zweite gekrümmte Bearbeitungsbahn mit einer in Richtung der Projektionsachse stetig zunehmenden oder stetig abnehmenden Bearbeitungshöhenkomponente zu bestimmen.

In einer Ausführungsvariante weist die erste Bearbeitungsbahn in Richtung der Projektionsachse eine stetige Höhenänderungskomponente auf, der Schaltkreis ist eingerichtet, Teilabschnitte der transformierten zweiten gekrümmten Bearbeitungsbahn mit einer in Richtung der Projektionsachse jeweils konstanten Bahnabschnittsbearbeitungshöhe zu bestimmen, und eine Erzeugung des gepulsten Laserstrahls durch die Laserquelle zu unterbinden, während die Fokussieroptik die Bahnabschnittsbearbeitungshöhe zweier in Richtung der Projektionsachse benachbarter Teilabschnitte der transformierten zweiten gekrümmten Bearbeitungsbahn verstellt.

In einer Ausführungsvariante weisen Teilabschnitte der ersten Bearbeitungsbahn in Richtung der Projektionsachse jeweils eine konstante Bahnabschnittsbearbeitungshöhe auf, und der Schaltkreis ist eingerichtet, die transformierte zweite gekrümmte Bearbeitungsbahn mit einer in Richtung der Projektionsachse stetig zunehmenden oder stetig abnehmenden Bearbeitungshöhenkomponente zu bestimmen.

In einer Ausführungsvariante weisen Teilabschnitte der ersten Bearbeitungsbahn in Richtung der Projektionsachse jeweils eine konstante Bahnabschnittsbearbeitungshöhe auf, und der Schaltkreis ist eingerichtet, Teilabschnitte der transformierten zweiten gekrümmten Bearbeitungsbahn mit einer in Richtung der Projektionsachse jeweils konstanten Bahnabschnittsbearbeitungshöhe zu bestimmen, und eine Erzeugung des gepulsten Laserstrahls durch die Laserquelle zu unterbinden, während die Fokussieroptik die Bahnabschnittsbearbeitungshöhe zweier in Richtung der Projektionsachse benachbarter Teilabschnitte der transformierten zweiten gekrümmten Bearbeitungsbahn verstellt.

In einer Ausführungsvariante ist der Schaltkreis eingerichtet, die transformierte zweite gekrümmte Bearbeitungsbahn mit einer sich in Richtung der Projektionsachse abwechselnd zunehmenden und abnehmenden Bearbeitungshöhenkomponente zu bestimmen.

In einer Ausführungsvariante umfasst die erste Bearbeitungsbahn Bahnabschnitte mit mindestens einer der folgenden Formen: einen kreisrunden Bahnabschnitt, einen elliptischen Bahnabschnitt, einen parabolischen Bahnabschnitt, einen hyperbolischen Bahnabschnitt, einen spiralförmigen Bahnabschnitt, oder einen Spline-Bahnabschnitt.

In einer Ausführungsvariante ist der Schaltkreis eingerichtet, die transformierte zweite gekrümmte Bearbeitungsbahn ausgehend von der ersten gekrümmten Bearbeitungsbahn zu bestimmen, durch Durchführung mindestens eine der folgenden Operationen: Strecken von ersten Bereichen der ersten gekrümmten Bearbeitungsbahn, Stauchen von zweiten Bereichen der ersten gekrümmten Bearbeitungsbahn, und/oder Unterbrechen von dritten Bereichen der ersten gekrümmten Bearbeitungsbahn.

In einer Ausführungsvariante ist der Schaltkreis eingerichtet, das Scannersystem zum Schneiden eines durch zwei Schnittflächen gebildeten zur Zentrumsachse des Patienteninterfaces symmetrischen Lentikels im Augengewebe zu steuern, und bei der Verkippung des Auges zur Zentrumsachse des Patienteninterfaces, einen Scheitelpunkt eines verkippten Lentikels zu ermitteln, welches durch eine der Verkippung des Auges entsprechende Mitverkippung des Lentikels bestimmt ist, transformierte dritte gekrümmte Bearbeitungsbahnen zu bestimmen, die um den Scheitelpunkt herum verlaufen und Bearbeitungspunkte auf den Schnittflächen des verkippten Lentikels bestimmen, und das Scannersystem derart zu steuern, dass der gepulste Laserstrahl auf Bearbeitungspunkte auf den transformierten dritten gekrümmten Bearbeitungsbahnen gelenkt wird.

In einer Ausführungsvariante weist das Patienteninterface einen zur Zentrumsachse symmetrischen, gewölbten Innenraum zur Aufnahme eines Hornhautbereichs des Auges auf.

In einer Ausführungsvariante weist das Patienteninterface einen zur Zentrumsachse symmetrischen, planaren Innenraum zur Aufnahme eines Hornhautbereichs des Auges auf.

In einer Ausführungsvariante umfasst die ophthalmologische Vorrichtung eine Messvorrichtung, die eingerichtet ist, Referenzstrukturen und/oder Referenzmarkierungen im oder auf dem Augengewebe zu erfassen, und der Schaltkreis ist eingerichtet, die Verkippung des Auges zur Zentrumsachse des Patienteninterfaces aufgrund der durch die Messvorrichtung erfassten Referenzstrukturen und/oder Referenzmarkierungen zu bestimmen.

In einer Ausführungsvariante ist der Schaltkreis eingerichtet, die Verkippung des Auges zur Zentrumsachse des Patienteninterfaces durch Ermitteln einer Verschiebung des Hornhautbereichs des Auges bezüglich einer Kontaktfläche des gewölbten Innenraums des Patienteninterfaces zu bestimmen.

In einer Ausführungsvariante umfasst die ophthalmologische Vorrichtung eines oder mehrere Ansaugelemente, welche eingerichtet sind, das Patienteninterface am Auge zu fixieren.

In einer Ausführungsvariante umfasst das Scannersystem eine erste Scannvorrichtung, die eingerichtet ist, den gepulsten Laserstrahl im Augengewebe mit einer Vorschubgeschwindigkeit entlang einer Vorschublinie auf der Bearbeitungsbahn zu lenken, und das Scannersystem umfasst eine zweite Scannvorrichtung, die eingerichtet ist, den gepulsten Laserstrahl im Augengewebe mit einer bezüglich der Vorschubgeschwindigkeit höheren Abtastgeschwindigkeit entlang einer quer zur Vorschublinie verlaufenden Abtastlinie auf der Bearbeitungsbahn zu lenken.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt ein Blockdiagramm, das schematisch eine ophthalmologische Vorrichtung zur Bearbeitung von Augengewebe mit einem gepulsten Laserstrahl illustriert, welche mittels eines Patienteninterfaces am Auge fixiert ist.
- Figur 2:: zeigt ein Blockdiagramm, das schematisch eine ophthalmologische Vorrichtung zur Bearbeitung von Augengewebe mit einem gepulsten Laserstrahl illustriert, welche mittels eines Patienteninterfaces am Auge fixiert ist, wobei das Auge zur Zentrumsachse des Patienteninterfaces verkippt ist.
- Figur 3:: zeigt eine schematische Querschnittsansicht eines Patienteninterfaces der ophthalmologischen Vorrichtung mit einem gewölbten Innenraum zur Aufnahme eines Hornhautbereichs des Auges und Ansaugelementen zur Fixierung des Patienteninterfaces am Auge.
- Figur 4:: zeigt eine schematische Querschnittsansicht einer Schnittfläche in einem Augengewebebereich, der an eine Kontaktfläche des Patienteninterfaces angrenzt und im gewölbten Innenraum des Patienteninterfaces unverkippt zur Zentrumsachse des Patienteninterfaces angeordnet ist.
- Figur 5:: zeigt eine schematische Querschnittsansicht einer Schnittfläche in einem Augengewebebereich, der an eine Kontaktfläche des Patienteninterfaces angrenzt und im gewölbten Innenraum des Patienteninterfaces verkippt zur Zentrumsachse des Patienteninterfaces angeordnet ist.
- Figur 6:: zeigt eine schematische Querschnittsansicht einer um die Projektionsachse einer Fokussieroptik herumverlaufenden, gekrümmten Bearbeitungsbahn zum Schneiden einer Schnittfläche in einem Augengewebebereich, der an eine Kontaktfläche des Patienteninterfaces angrenzt und im gewölbten Innenraum des Patienteninterfaces unverkippt zur Zentrumsachse des Patienteninterfaces angeordnet ist.
- Figur 7:: zeigt eine schematische Querschnittsansicht einer um einen verschobenen Scheitelpunkt einer verkippten Schnittfläche herumverlaufenden, gekrümmten Bearbeitungsbahn mit Unterbrechungen zum Schneiden einer Schnittfläche in einem Augengewebebereich, der an eine Kontaktfläche des Patienteninterfaces angrenzt und im gewölbten Innenraum des Patienteninterfaces verkippt zur Zentrumsachse des Patienteninterfaces angeordnet ist.
- Figur 8:: zeigt eine schematische Aufsicht einer um einen Scheitelpunkt einer verkippten Schnittfläche herumverlaufenden, gekrümmten Bearbeitungsbahn mit Unterbrechungen zum Schneiden einer Schnittfläche in einem Augengewebebereich, der zur Zentrumsachse des Patienteninterfaces verkippt ist.
- Figur 9:: zeigt eine schematische Querschnittsansicht einer um einen Scheitelpunkt einer verkippten Schnittfläche herumverlaufenden, gekrümmten Bearbeitungsbahn mit Unterbrechungen zum Schneiden einer Schnittfläche in einem Augengewebebereich, der zur Zentrumsachse des Patienteninterfaces verkippt ist.
- Figur 10:: zeigt eine schematische Aufsicht einen Umriss einer Schnittfläche respektive einer dafür vorgesehenen Bearbeitungsbahn sowie den Umriss der Schnittfläche respektive der Bearbeitungsbahn bei einer Verkippung des Auges zur Zentrumsachse des Patienteninterfaces.
- Figur 11:: zeigt eine schematische Aufsicht einer um einen Scheitelpunkt einer verkippten Schnittfläche herumverlaufenden, gekrümmten und verzerrten Bearbeitungsbahn zum Schneiden einer Schnittfläche in einem Augengewebebereich, der zur Zentrumsachse des Patienteninterfaces verkippt ist.
- Figur 12:: zeigt ein Flussdiagramm, das eine mögliche Abfolge von Schritten zur Ermittlung eines Scheitelpunkts einer verkippten Schnittfläche und einer entsprechend transformierten Bearbeitungsbahn bei einer Verkippung des Auges zur Zentrumsachse des Patienteninterfaces illustriert.

### Wege zur Ausführung der Erfindung

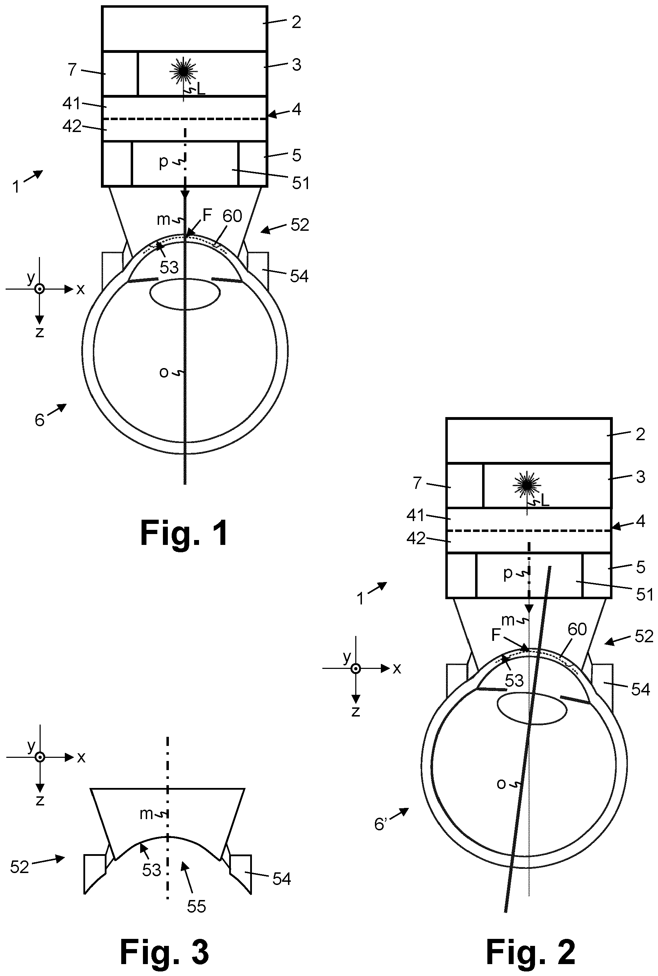

In den Figuren 1 und 2, bezieht sich das Bezugszeichen 1 jeweils auf eine ophthalmologische Vorrichtung zum Bearbeiten von Augengewebe 60 mittels Laserpulsen, beispielsweise die Cornea oder anderes Gewebe eines Auges 6.

Wie in den Figuren 1 und 2 schematisch dargestellt ist, umfasst die ophthalmologische Vorrichtung 1 eine Laserquelle 3, ein Scannersystem 4, und einen Applikationskopf 5 mit einer Fokussieroptik 51 und einem Patienteninterface 52.

Die Laserquelle 3 ist eingerichtet, einen gepulsten Laserstrahl L zu erzeugen. Die Laserquelle 3 umfasst insbesondere einen Femtosekundenlaser zur Erzeugung von Femtosekundenlaserpulsen, die Pulsbreiten von typisch 10fs bis 1000fs (1fs=10⁻¹⁵s) aufweisen. Die Laserquelle 3 ist in einem separaten oder in einem mit der Fokussieroptik 51 gemeinsamen Gehäuse angeordnet.

Das Scannersystem 4 ist eingerichtet, den von der Laserquelle 3 gelieferten gepulsten Laserstrahl L über die Fokussieroptik 51 im Augengewebe 60 auf Bearbeitungspunkte F auf einer Bearbeitungsbahn t, t' (Trajektorien t und t') zu lenken. Wie in den Figuren 1 und 2 schematisch dargestellt ist, umfasst das Scannersystem 4 mehrere Scannvorrichtungen: eine erste Scannvorrichtung 41 (Fast-Scan-Modul) und eine zweite Scannvorrichtung 42 (Slow-Scan-Modul). In einer Ausführungsvariante umfasst das Scannersystem 4 einen Divergenzmodulator zur Modulation der Fokustiefe respektive der Bearbeitungshöhe in Projektionsrichtung entlang der Projektionsachse p.

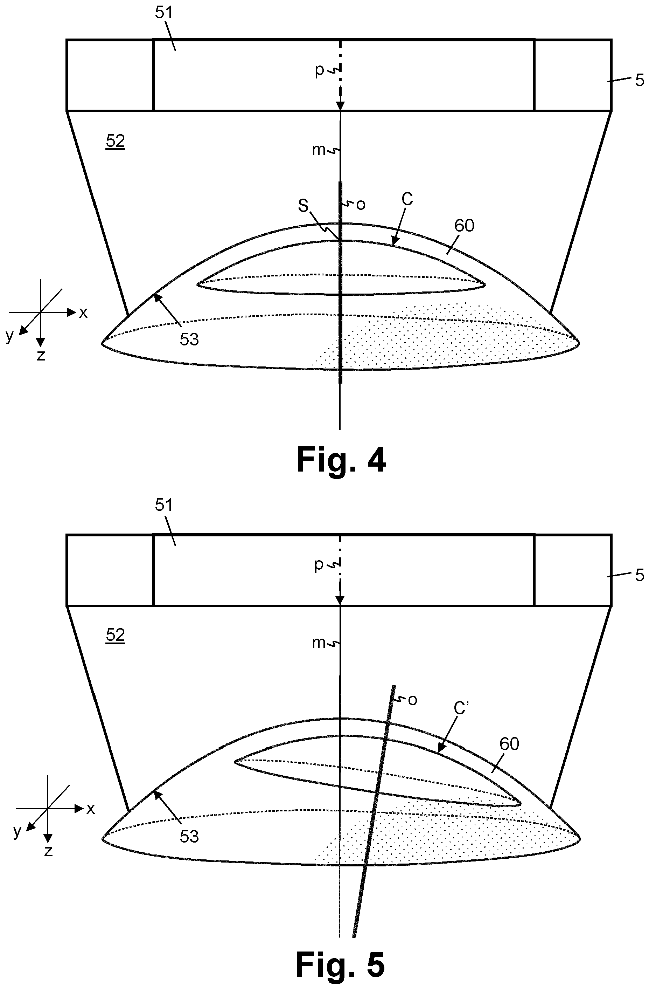

Die der Fokussieroptik 51 vorgelagerte Scannvorrichtung 42 (Slow-Scan-Modul) ist eingerichtet, das Augengewebe 60 mit dem gepulsten Laserstrahl L respektive dessen Laserpulsen in einer x/y-Bearbeitungsebene mit einer Vorschubgeschwindigkeit entlang einer Vorschublinie auf der Bearbeitungsbahn t, t' zu lenken, wie beispielhaft in den Figuren 5, 6, 7, 8 und 10 illustriert ist. Die Scannvorrichtung 42 ist als mechanischer Scanner ausgeführt, der die Fokussieroptik 51 mittels einem oder mehreren Bewegungstreibern über ein Bearbeitungsgebiet entlang der Bearbeitungsbahn t, t' abfährt, so dass der Fokus der Fokussieroptik 51 in der x/y-Bearbeitungsebene entlang der Bearbeitungsbahn t, t' geführt wird, oder die Scannvorrichtung 42 ist strahlablenkend ausgeführt und umfasst einen oder zwei um jeweils eine oder zwei Achsen bewegliche Ablenkspiegel zum Ablenken des gepulsten Laserstrahls L respektive der Laserpulse in der x/y-Bearbeitungsebene entlang der Bearbeitungsbahn t, t'. Die strahlablenkende Scannvorrichtung 42 ist als frei adressierbarer Scanner ausgeführt und umfasst beispielsweise einen Galvanoscanner oder einen mit einem Piezo angetriebenen Scanner.

In einer Ausführungsvariante ist die der Scannvorrichtung 42 vorgeschaltete Scannvorrichtung 41 (als Fast-Scan-Modul) eingerichtet, das Augengewebe 60 mit dem gepulsten Laserstrahl L respektive dessen Laserpulsen in der x/y-Bearbeitungsebene mit einer bezüglich der Vorschubgeschwindigkeit höheren Abtastgeschwindigkeit entlang einer quer zur Vorschublinie verlaufenden Abtastlinie zu lenken. Die beiden Scannvorrichtungen 41 und 42 sind so eingerichtet und gekoppelt, dass die entlang der Abtastlinie verlaufende Scannbewegung der Scannvorrichtung 41 der Vorschublinie der Scannvorrichtung 42 überlagert ist. Die Scannvorrichtung 41 umfasst einen oder mehrere bewegliche Ablenkspiegel, beispielsweise ein rotierender Polygonspiegel (Polygonscanner), ein oder mehrere resonante Spiegel (resonanter Scanner) oder oszillierende Spiegel (oszillierender Scanner), die beispielsweise Piezo-angetrieben sind (Piezo-Scanner), oder MEM Scanner (Micro Electro-Mechanical), oder die Scannvorrichtung 41 umfasst einen AOM Scanner (Acusto-optischer Modulator) oder einen EOM Scanner (Electro-optischer Modulator). Die Scannvorrichtung 41 weist eine, beispielsweise mehrfach, höhere Scanngeschwindigkeit (Abtastgeschwindigkeit) auf als die nachgelagerte Scannvorrichtung 42 (Vorschubgeschwindigkeit). Um Missverständnisse zu vermeiden, soll an dieser Stelle angeführt werden, dass bei der Ausführungsvariante, in welcher die als Fast-Scan-Modul eingerichtete Scannvorrichtung 41 Abtastlinien erzeugt, die quer zur Vorschublinie der Scannvorrichtung 42 verlaufen, die Vorschublinie der nachgelagerten Scannvorrichtung 42 der Bearbeitungsbahn t, t' entspricht, wobei die quer verlaufenden Abtastlinien der vorgelagerten Scannvorrichtung 41 Zwischenräume zwischen den Bearbeitungsbahn t, t' bearbeiten.

Wie in den Figuren 1, 2 und 4-7 schematisch dargestellt ist, umfasst der Applikationskopf 5 eine Fokussieroptik 51 und ein Patienteninterface 52. Der Applikationskopf 5 ist mittels des Patienteninterfaces 52 auf das Auge 6 aufsetzbar und am Auge 6 fixierbar. Der Einfachheit halber ist in den Figuren 4-7 jeweils nur der am Auge 6 angebrachte und fixierte Applikationskopf 5 dargestellt, ohne die weiteren Module der ophthalmologische Vorrichtung 1 wiederzugeben, die in den Figuren 1 und 2 dargestellt sind.

Die Fokussieroptik 51 ist eingerichtet, den gepulsten Laserstrahl L respektive dessen Laserpulse für die punktuelle Gewebeauflösung auf die Bearbeitungspunkte F im Innern des Augengewebes 60 zu fokussieren. Die Fokussieroptik 51 umfasst ein Linsensystem, mit einer oder mehreren optischen Linsen. Abhängig von der Ausführungsvariante umfasst die Fokussieroptik 51 eine oder mehrere bewegliche Linsen und/oder oder einen Antrieb zum Bewegen der gesamten Fokussieroptik 51 zum Ein- und Verstellen der Fokustiefe respektive der Bearbeitungshöhe in Projektionsrichtung entlang der Projektionsachse p. In einer weiteren Ausführungsvariante ist im Strahlengang zwischen der Laserquelle 3 und dem Scannersystem 4 ein Divergenzmodulator vorgesehen.

Für das Bearbeiten und Schneiden von Schnittflächen C, C', welche eine zur Tiefenkomponente in Projektionsrichtung entlang der Projektionsachse p vergleichsweise grössere laterale Komponente in zur Projektionsrichtung normalen x/y-Bearbeitungsebene aufweisen, ist das Scannersystem 4 eingerichtet, die Bearbeitungspunkte F, auf die die Laserpulse fokussiert werden, mit einer bezüglich der Fokusverstellgeschwindigkeit der Fokussieroptik 51 höheren Scanngeschwindigkeit auf der Bearbeitungsbahn t, t' zu verschieben.

Wie in den Figuren 1 bis 7 schematisch dargestellt ist, umfasst das Patienteninterface 52 eines oder mehrere Haltelemente, z.B. Ansaugelemente 54, zur Fixierung des Patienteninterfaces 52 am Auge 6. Das Ansaugelement 54 ist beispielsweise als Saugring ausgeführt. Wie in der Figur 3 schematisch dargestellt ist, weist das Patienteninterface 52 einen gewölbten Innenraum 55 zur Aufnahme eines Hornhautbereichs 60 des Auges 6 auf. Der gewölbte Innenraum 55 ist symmetrisch zur Zentrumsachse m des Patienteninterfaces 52. Der gewölbte Innenraum 55 wird durch eine Kontaktfläche 53 des Patienteninterfaces 52 gebildet. Im applizierten Zustand, kontaktiert die Kontaktfläche 53 des Patienteninterfaces 52 die äussere Oberfläche der Hornhaut 60 des Auges 6. Abhängig von der Ausführungsvariante ist das Patienteninterface 52 fest oder entfernbar mit der Fokussieroptik 51 verbunden.

Wie in den Figuren 1 und 2 schematisch dargestellt ist, umfasst die ophthalmologische Vorrichtung 1 einen Schaltkreis 2 zur Steuerung der Laserquelle 3, der optischen Funktionsmodule des Scannersystems 4, der Fokussieroptik 51 und der nachfolgend beschriebenen Messvorrichtung 7. Der Schaltkreis 2 realisiert eine programmierbare Steuervorrichtung und umfasst beispielsweise ein oder mehrere Prozessoren mit Programm- und Datenspeicher sowie programmierte Softwaremodule zur Steuerung der Prozessoren, und/oder andere programmierbare Schaltungen oder Logikeinheiten wie ASICs (Application Specific Integrated Circuit).

Wie in den Figuren 1 und 2 schematisch dargestellt ist, umfasst die ophthalmologische Vorrichtung 1 eine Messvorrichtung 7, beispielsweise eine bildgebende Messvorrichtung, z.B. ein Videosensor, oder eine interferometrische Messvorrichtung, z.B. ein OCT System (Optical Coherence Tomography), die optisch in den Strahlengang des Scannersystems 4 zur Fokussieroptik 51 eingekoppelt ist. Die Messvorrichtung 7 ist eingerichtet über den Strahlengang Referenzstrukturen und/oder Referenzmarkierungen im oder auf dem Augengewebe 60 zu erfassen. Die Messvorrichtung 7 ist mit dem Schaltkreis 2 verbunden. Der Schaltkreis 2 ist eingerichtet, aufgrund der durch die Messvorrichtung 7 erfassten Referenzstrukturen und/oder Referenzmarkierungen Verkippungen des Auges 6 bezüglich des Patienteninterfaces 52, insbesondere Verkippungen des Auges 6 bezüglich der Zentrumsachse m des Patienteninterfaces 52 zu detektieren. In einer Ausführungsvariante ist der Schaltkreis 2 eingerichtet, die Verkippungen des Auges 6 bezüglich des Patienteninterfaces 52 durch Ermitteln von Verschiebungen des Hornhautbereichs 60 des Auges 6 respektive der erfassten Referenzstrukturen und/oder Referenzmarkierungen bezüglich der Kontaktfläche 53 im gewölbten Innenraum des Patienteninterfaces 52 zu bestimmen.

In den Figuren 1, 4 und 6 sind Situationen dargestellt, in denen das Auge 6 koaxial zur Zentrumsachse m des Patienteninterfaces 52 ausgerichtet ist. Mit anderen Worten, die durch den Scheitelpunkt des Auges 6 verlaufende Augenachse o verläuft im Wesentlichen koaxial zur Zentrumsachse m des Patienteninterfaces 52. Bei idealer Anordnung und Ausrichtung des Patienteninterfaces 52 zur Fokussieroptik 51, verläuft die Zentrumsachse m des Patienteninterfaces 52 koaxial zur Projektionsachse p der Fokussieroptik 51.

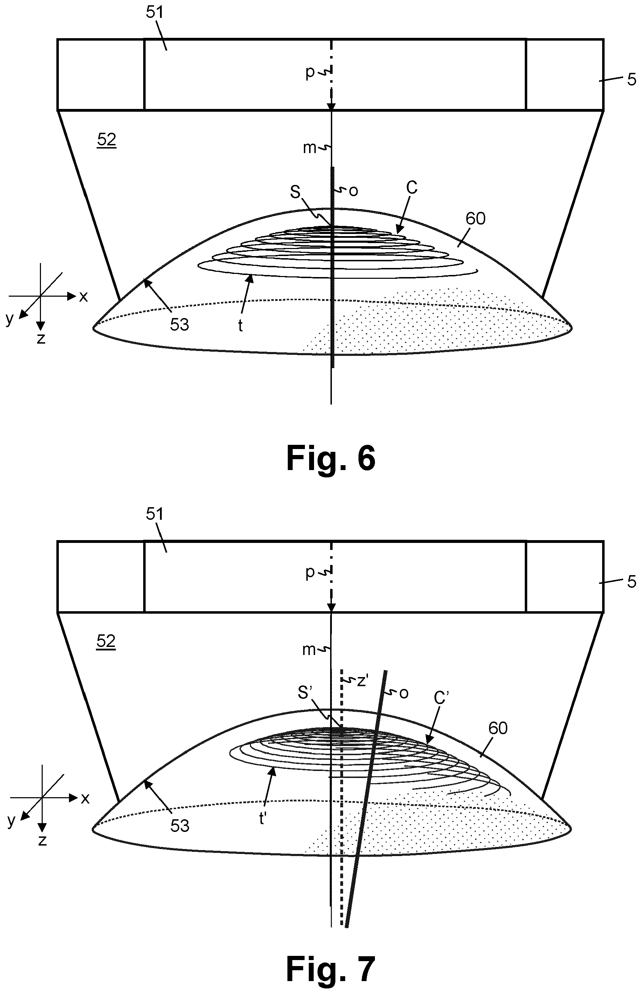

In den Figuren 4 und 6 sind Schnittflächen C im Augengewebe 60, insbesondere in der Hornhaut des Auges 6, dargestellt, welche symmetrisch zur Zentrumsachse m des Patienteninterfaces 52 sind, wenn die Zentrumsachse m des Patienteninterfaces 52 koaxial zur Projektionsachse p der Fokussieroptik 51 ausgerichtet ist. Bei koaxialer Ausrichtung des Auges 6 zur Zentrumsachse m des Patienteninterfaces 5, sind die Schnittflächen C im Augengewebe 60 auch koaxial zur Augenachse o, die durch den Scheitelpunkt des Auges 6 verläuft. In einer Ausführungsvariante umfasst die Schnittfläche C zwei Teilschnittflächen, welche im Augengewebe 60 ein zur Zentrumsachse m des Patienteninterfaces 52 symmetrisches Lentikel bestimmen respektive bilden. In den nachfolgenden Ausführungen zur Schnittfläche C und zu einer verkippten Schnittfläche C' kann diese Bildung des Lentikels durch die Teilschnittflächen der Schnittfläche C respektive durch die Teilschnittflächen der verkippten Schnittfläche C' stets mitgelesen werden, auch wenn darauf nicht weiter eingegangen wird.

Im Beispiel der Figur 6, ist eine Bearbeitungsbahn t zum Schneiden der Schnittfläche C illustriert. Die Bearbeitungsbahn t zum Schneiden der Schnittfläche C ist gekrümmt und verläuft um die Projektionsachse p der Fokussieroptik 51 herum. Im Beispiel der Figur 6, ist die Bearbeitungsbahn t eine spiralförmige Bahn, welche ausgehend vom Scheitelpunkt S der Schnittfläche C auf der Schnittfläche C verläuft und eine stetige Steigung, mit einer monotonen Änderung der Bearbeitungshöhe in Richtung der Projektionsachse p aufweist. Dabei ist der Scheitelpunkt S der Schnittfläche C der höchstgelegene Punkt der Schnittfläche C hinsichtlich der Richtung der Projektionsachse p. Die Bearbeitungsbahn t weist überdies eine sich mit abnehmender Bearbeitungshöhe respektive zunehmender Bearbeitungstiefe vergrössernde Distanz zur Projektionsachse p auf. Der Fachmann wird verstehen, dass der Schneidvorgang durch Abtasten der Bearbeitungsbahn t ausgehend von ihrem tiefsten Punkt im Auge 6 zu Ihrem Scheitelpunkt S oder umgekehrt ausgeführt werden kann. Abhängig von der Ausführung und Konfiguration umfasst die Bearbeitungsbahn t einen oder mehrere voneinander getrennte oder zusammenhängende Bahnabschnitte. Die Bahnabschnitte weisen eine kreisrunde, eine elliptische, eine parabolische, eine hyperbolische, eine spiralförmige und/oder eine Spline Form auf. Die Bearbeitungsbahn t zum Schneiden einer Schnittfläche C wird durch gespeicherte Steuerdaten definiert. Aufgrund der Steuerdaten steuert der Schaltkreis 2 die Laserquelle 3, das Scannersystem 4 und/oder die Fokussiervorrichtung 51, um die durch die Steuerdaten definierte Schnittfläche durch Abtasten der Bearbeitungsbahn t mittels des gepulsten Laserstrahls L zu schneiden.

In den Figuren 2, 5 und 7 sind Situationen dargestellt, in denen das Auge 6 zur Zentrumsachse m des Patienteninterfaces 52 verkippt ist. Mit anderen Worten, die durch den Scheitelpunkt des Auges 6 verlaufende Augenachse o verläuft schräg oder quer zur Zentrumsachse m des Patienteninterfaces 52. Bei verkippter Anordnung und Ausrichtung des Auges 6 zum Patienteninterface 52, ist das Auge 6 entsprechend auch zur Projektionsachse p der Fokussieroptik 51 verkippt. In den Figuren 5 und 7 sind verkippte Schnittflächen C' im Augengewebe 60 dargestellt, welche mit der Verkippung des Auges 6 mitverkippt sind und somit symmetrisch zur Augenachse o sind. Aufgrund des gewölbten Innenraums 55 respektive der entsprechend gewölbten Kontaktfläche 53 des Patientinterfaces 52, welche bei appliziertem Patienteninterface 52 die Cornea 60 des Auges 6 kontaktiert, entspricht eine Verkippung des Auges 6 zur Zentrumsachse m des Patienteninterfaces 52 im Wesentlichen einer Verschiebung der äusseren Oberfläche der Cornea 60 bezüglich der Kontaktfläche 53 des Patienteninterfaces 52.

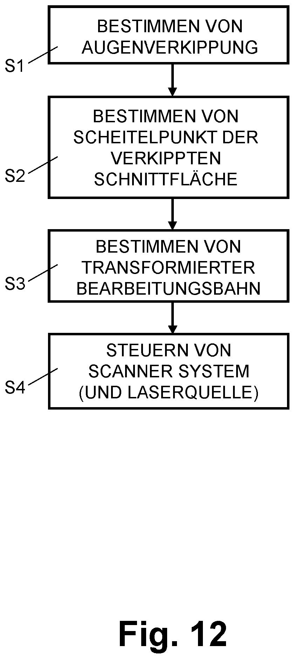

In den folgenden Abschnitten werden mit Bezug zu der Figur 12 Schritte beschrieben, die durch den Schaltkreis 2 zur Anpassung von Bearbeitungsbahnen t für die Erzeugung von Schnittflächen C, C' im Augengewebe 60 durchgeführt werden, wenn das Auge 6 zur Zentrumsachse m des Patienteninterfaces 52 verkippt ist.

Im Schritt S1, bezieht der Schaltkreis 2 die von der Messvorrichtung 7 aktuell erfassten Referenzstrukturen und/oder Referenzmarkierungen im oder auf dem Augengewebe 60, und ermittelt eine allfällige Verkippung des Auges 6 bezüglich der Zentrumsachse m des Patienteninterfaces 52. Dazu ermittelt der Schaltkreis 2 Verschiebungen und/oder Verdrehungen der aktuell erfassten Referenzstrukturen und/oder Referenzmarkierungen bezüglich der Lage und Orientierung von gespeicherten Referenzstrukturen und/oder Referenzmarkierungen, die in einem zuvor erfolgten Vorbereitungsschritt für das betreffende Auge 6 bei unverkippter Ausrichtung des Auges 6 bezüglich der Zentrumsachse m des Patienteninterfaces 52 oder eines Referenzsystems erfasst wurden. Alternativ wird die Verkippung des Auges 6 bezüglich der Zentrumsachse m des Patienteninterfaces 52 (ohne Messvorrichtung) durch den Benutzer bestimmt, der beispielsweise eine Verschiebebewegung im angedockten Zustand ausführt.

Bei einer Verkippung des Auges 6 zur Zentrumsachse m des Patienteninterfaces 52, bestimmt der Schaltkreis 2 im Schritt S2, ausgehend von den gespeicherten Steuerdaten für die Bearbeitungsbahn t der im Augengewebe 60 zu schneidenden Schnittfläche C, den Scheitelpunkt S' einer verkippten Schnittfläche C'. Die verkippte Schnittfläche C' ist durch eine Mitverkippung der Schnittfläche C definiert, welche der im Schritt S1 bestimmten Verkippung des Auges 6 entspricht. Mit anderen Worten, die verkippte Schnittfläche C' kann im verkippten Auge 6' geschnitten werden, um im Augengewebe 60 die durch die gespeicherten Steuerdaten im unverkippten Auge 6 definierte Schnittfläche C zu schneiden. Die Bestimmung des Scheitelpunkts S' der verkippten Schnittfläche C' erfolgt durch Ermitteln des hinsichtlich der Richtung der Projektionsachse p höchstgelegenen Punkts der verkippten Schnittfläche C' unter Verwendung und gemäss der im Schritt S1 bestimmten Verkippung des Auges 6. Abhängig von der Oberflächenkrümmung der Schnittfläche C respektive der verkippten Schnittfläche C' hinsichtlich der Oberflächenkrümmung der Wölbung des Patienteninterfaces 52 (respektive der durch diese Wölbung angepassten Augenoberfläche des Auges 6), verschiebt sich der Scheitelpunkt S' der verkippten Schnittfläche C' bezüglich des Scheitelpunkts S der unverkippten Schnittfläche C, wie im Beispiel der Figur 7 ersichtlich ist. Wenn die Schnittfläche C respektive die verkippte Schnittfläche C' und das Auge 6 äquidistante Oberflächen aufweisen, haben der Scheitelpunkt S' der verkippten Schnittfläche C' und der Scheitelpunkt S der unverkippten Schnittfläche C dieselbe Lage, das heisst bei äquidistanten Oberflächen von Auge 6 und Schnittfläche C bewirkt die Verkippung des Auges 6 und bei der mitverkippten Schnittfläche C' keine Verschiebung des Scheitelpunkts (S=S').

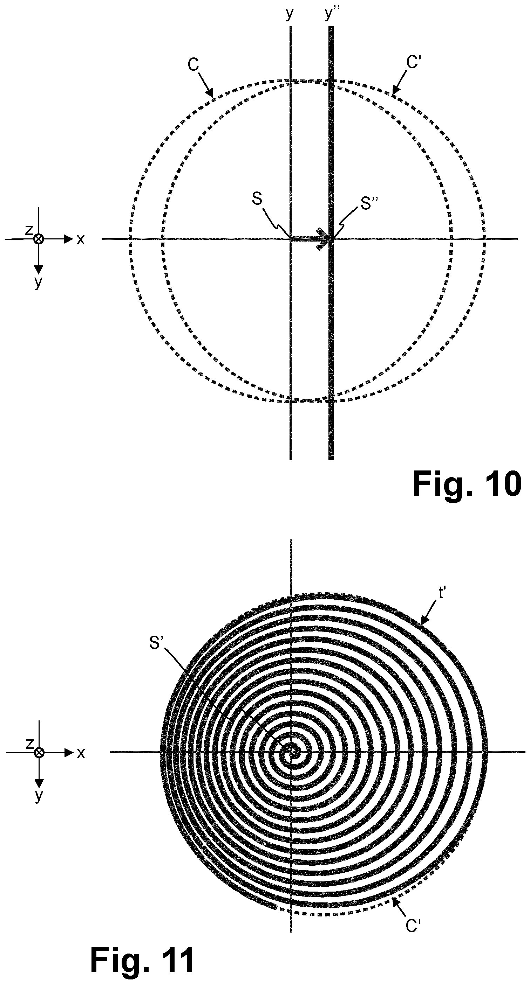

Figur 10 illustriert die unverkippte Schnittfläche C und die verkippte Schnittfläche C' in der Aufsicht. Wie in der Figur 10 ersichtlich ist, verschiebt sich bei der verkippten Schnittfläche C' die Querachse y" bezüglich der Querachse y der Schnittfläche C im unverkippten Auge 6. Dabei verschiebt sich der Scheitelpunkt S der unverkippten Schnittfläche C zum verkippten Scheitelpunkt S". Dieser verkippte Scheitelpunkt S" entspricht jedoch in der Regel nicht dem Scheitelpunkt S' der verkippten Schnittfläche C'. Abhängig von der Oberflächenkrümmung der Schnittfläche C respektive der verkippten Schnittfläche C' liegt der Scheitelpunkt S' der verkippten Schnittfläche C' an derselben Position wie der Scheitelpunkt S der unverkippten Schnittfläche C (S'=S), an der Position des verkippten Scheitelpunkts S" (S'=S") oder an einer Position, die zwischen diesen beiden Punkten liegt (S'=[S... S"]).

Im Schritt S3, bestimmt der Schaltkreis 2 eine transformierte Bearbeitungsbahn t' zum Schneiden der verkippten Schnittfläche C'. Die Figuren 7, 8, 9 und 11 zeigen Beispiele von transformierten Bearbeitungsbahnen t' zum Schneiden der verkippten Schnittfläche C'. Wie in den Beispielen der Figuren 7, 8, 9 und 11 ersichtlich ist, ist die transformierte Bearbeitungsbahn t' jeweils gekrümmt und verläuft um den Scheitelpunkt S' der verkippten Schnittfläche C' herum. Der Schaltkreis 2 ist eingerichtet, die Transformation respektive die Erzeugung der transformierten Bearbeitungsbahnen t' zum Schneiden der verkippten Schnittfläche C' unter Berücksichtigung der Dynamik des Scannersystems 4 (insbesondere Scanngeschwindigkeit und Vorschubgeschwindigkeit des Scannersystems 4) auszuführen, so dass die transformierten Bearbeitungsbahnen t' durch die Fokussieroptik 51, ohne Überschreitung der maximalen Fokusverstellgeschwindigkeit der Fokussieroptik 51, abfahrbar sind.

Wie die ursprüngliche Bearbeitungsbahn t zum Schneiden der unverkippten Schnittfläche C, ist die durch den Schaltkreis 2 transformierte Bearbeitungsbahn t' zum Schneiden der verkippten Schnittfläche C' durch das Scannersystem 4 mit dem gepulsten Laserstrahl L in der normal zur Projektionsachse p verlaufenden x/y-Bearbeitungsebene mit der Scanngeschwindigkeit abtastbar, die höher ist als die Fokusverstellgeschwindigkeit der Fokussieroptik 51, und weist Höhenänderungen in Richtung der Projektionsachse p auf, welche durch die Fokussieroptik 51 durch stetige und vorzugsweise kontinuierliche Verstellung der Bearbeitungshöhe (Fokustiefe des gepulsten Laserstrahls L) in Richtung der Projektionsachse p mit der Fokusverstellgeschwindigkeit abfahrbar ist. Abhängig von der Bearbeitungsvariante, ist die Bearbeitungshöhe entlang der transformierten Bearbeitungsbahn t' durch die Fokussieroptik 51 mit einer stetig und kontinuierlich zunehmenden oder einer stetig und kontinuierlich abnehmenden Bearbeitungshöhenkomponente synchron zum Scannersystem 4 respektive zum Scannen, d.h. zum Abtasten der Bearbeitungspunkte entlang der transformierten Bearbeitungsbahn t', durch das Scannersystem 4 einstellbar. In einer Ausführungsvariante bestimmt der Schaltkreis 2 die transformierte Bearbeitungsbahn t' zum Schneiden der verkippten Schnittfläche C' mit einer sich in Richtung der Projektionsachse p abwechselnd zunehmenden und abnehmenden Bearbeitungshöhenkomponente. In einer Ausführungsvariante bestimmt der Schaltkreis 2 einzelne Bahnabschnitte der transformierten Bearbeitungsbahn t' mit einer sich in Richtung der Projektionsachse p abwechselnd zunehmenden und abnehmenden Bearbeitungshöhenkomponente und/oder mit einer gleichbleibenden (konstanten) Bearbeitungshöhe.

Die transformierte Bearbeitungsbahn t' umfasst einen oder mehrere voneinander getrennte oder zusammenhängende transformierte Bahnabschnitte. Die transformierte Bearbeitungsbahn t' bestimmt Bearbeitungspunkte auf der verkippten Schnittfläche C' zum Schneiden der verkippten Schnittfläche C'. Der Schaltkreis 2 bestimmt die transformierte Bearbeitungsbahn t' ausgehend von der durch die gespeicherten Steuerdaten definierten ursprünglichen Bearbeitungsbahn t durch eine oder mehrere der folgenden Transformationsschritte: Verzerren von einem oder mehreren Bereichen oder der gesamten ursprünglichen Bearbeitungsbahn t durch Strecken von einem oder mehreren Bereichen oder der gesamten ursprünglichen Bearbeitungsbahn t, oder durch Stauchen von einem oder mehreren Bereichen oder der gesamten ursprünglichen Bearbeitungsbahn t, und/oder Unterbrechen von einem oder mehreren Bereichen der ursprünglichen Bearbeitungsbahn t oder der transformierten Bearbeitungsbahn t'.

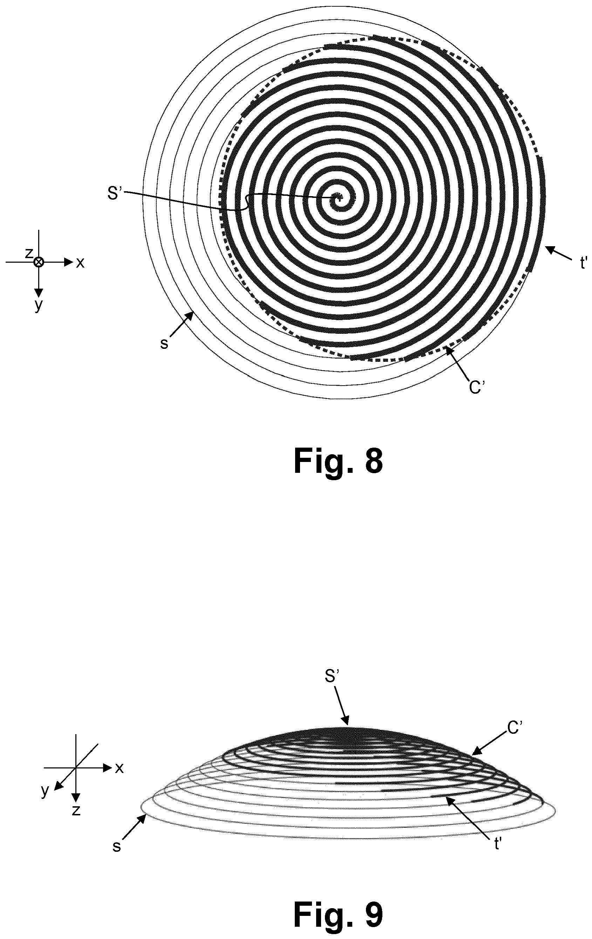

Die transformierte Bearbeitungsbahn t' im Beispiel der Figur 7 wird durch mehrere Transformationsschritte aus der Bearbeitungsbahn t der Figur 6 zum Schneiden der unverkippten Schnittfläche C erzeugt. Die Figur 8 illustriert in der Aufsicht die transformierte Bearbeitungsbahn t' der Figur 7 zum Schneiden der verkippten Schnittfläche C'. Wie in der Figur 8 ersichtlich ist, verläuft die transformierte Bearbeitungsbahn t' spiralförmig um den (verschobenen) Scheitelpunkt S' der verkippten Schnittfläche C'. Die fett markierten Bahnabschnitte stellen die durch den gepulsten Laserstrahl L abgetasteten Bearbeitungsbahn t' zum Schneiden der verkippten Schnittfläche dar. Die nicht fett markierten Bereiche auf der spiralförmigen Bahn s entsprechen Unterbrechungen, die nicht auf der verkippten Schnittfläche C' sind und nicht durch den gepulsten Laserstrahl L behandelt werden. Die Figur 9 illustriert die transformierte Bearbeitungsbahn t' der Figuren 7 und 8 in schematischer dreidimensionaler Ansicht. Wie aus den Figuren 6, 7 und 8 ersichtlich ist, erfolgt die Transformation gegebenenfalls durch Verschieben der ursprünglichen Bearbeitungsbahn t vom Scheitelpunkt S der unverkippten Schnittfläche C zum verschobenen Scheitelpunkt S' der verkippten Schnittfläche C' und andererseits durch Einführen von zusätzlichen Bahnen, z.B. in tieferen Bearbeitungstiefen in Richtung der Projektionsachse p in Fällen mit diesbezüglich hochgelagertem Scheitelpunkt S, S' (wie in den Beispielen der Figuren 7 und 9 gezeigt) oder in höheren Bearbeitungstiefen in Richtung der Projektionsachse p in Fällen mit diesbezüglich tiefgelagertem Scheitelpunkt S, S', und von Unterbrechungen auf der der spiralförmigen Bahn s.

Die transformierte Bearbeitungsbahn t' im Beispiel der Figur 11 wird durch eine Verzerrungs-Transformation (Stauchen und/oder Strecken) aus der Bearbeitungsbahn t zum Schneiden einer unverkippten Schnittfläche C erzeugt. Im Beispiel der Figur 11 wird die transformierte Bearbeitungsbahn t' einzig durch eine Verzerrung (Stauchen und/oder Strecken) der Bearbeitungsbahn t zum Schneiden der unverkippten Schnittfläche C in die transformierte Bearbeitungsbahn t' zum Schneiden zumindest eines Teilbereichs der verkippten Schnittfläche C' transformiert. Weitere ergänzende Teilbereiche der verkippten Schnittfläche C' werden gegebenenfalls mit zusätzlichen transformierten Bearbeitungsbahnen t' bearbeitet, welche Unterbrechungen aufweisen.

Im Schritt S4, steuert der Schaltkreis 2 das Scannersystem 4 so, dass der gepulste Laserstrahl L auf Bearbeitungspunkte F auf der transformierten Bearbeitungsbahn t' gelenkt wird. Der Schaltkreis 2 steuert das Scannersystem 4 so, dass der gepulste Laserstrahl L die transformierte Bearbeitungsbahn t' abtastet und die verkippte Schnittfläche C' schneidet. Im Fall von unterbrochenen Bahnabschnitten auf der transformierten Bearbeitungsbahn t', steuert der Schaltkreis 2 die Laserquelle 3 so, dass der gepulste Laserstrahl L bei Unterbrechungen der transformierten Bearbeitungsbahn t' ausgesetzt wird. Sobald die Verstellung der Fokussieroptik 51 in Richtung der Projektionsachse p die Bearbeitungshöhe und das Scannersystem 4 die x/y-Position (z.B. bezüglich der Projektionsachse p) des ersten Bearbeitungspunkts nach der Unterbrechung der transformierten Bearbeitungsbahn t' erreicht haben, steuert der Schaltkreis 2 die Laserquelle 3 so, dass der gepulste Laserstrahl L wieder aktiviert respektive durchgeschaltet wird und auf den betreffenden Bearbeitungspunkt F auf der transformierten Bearbeitungsbahn t' fokussiert wird. Bei Ausführungsvarianten respektive Bearbeitungsvarianten, bei denen Teilabschnitte der Bearbeitungsbahn t und/oder der transformierten Bearbeitungsbahn t' eine gleichbleibende (konstante) Bearbeitungshöhe aufweisen, steuert der Schaltkreis 2 die Fokussieroptik 51 so, dass sie die Bearbeitungshöhe auf den betreffenden Teilabschnitten unverändert lässt und danach, vor oder während der Bearbeitung des darauffolgenden Bahnabschnitts der Bearbeitungsbahn t und/oder der transformierten Bearbeitungsbahn t' die Bearbeitungshöhe verstellt.

## Patentansprüche

1. Ophthalmologische Vorrichtung (1) zum Bearbeiten von Augengewebe (60) eines Auges (6), umfassend eine Laserquelle (3), die eingerichtet ist, einen gepulsten Laserstrahl (L) zu erzeugen; einen Applikationskopf (5) mit einer Fokussieroptik (51) und einem Patienteninterface (52), wobei die Fokussieroptik (51) eine Projektionsachse (p) aufweist und eingerichtet ist, den gepulsten Laserstrahl (L) im Augengewebe (60) auf einen Bearbeitungspunkt zu fokussieren, und wobei das Patienteninterface (52) eine Zentrumsachse (m) aufweist und eingerichtet ist, den Applikationskopf (5) am Auge zu fixieren; ein Scannersystem (4), das eingerichtet ist, den gepulsten Laserstrahl (L) im Augengewebe (60) auf Bearbeitungspunkte auf einer Bearbeitungsbahn (t) zu lenken; und einen Schaltkreis (2), der eingerichtet ist, das Scannersystem (4) zum Schneiden einer zur Zentrumsachse (m) des Patienteninterfaces (52) symmetrischen Schnittfläche (C) im Augengewebe (60) zu steuern, wobei der gepulste Laserstrahl (L) auf Bearbeitungspunkte auf der Schnittfläche (C) auf einer ersten Bearbeitungsbahn (t) gelenkt wird,
**dadurch gekennzeichnet, dass** der Schaltkreis (2) überdies eingerichtet ist, bei einer Verkippung des Auges (6) zur Zentrumsachse (m) des Patienteninterfaces (52), einen Scheitelpunkt (S') einer verkippten Schnittfläche (C') zu ermitteln, welche durch eine der Verkippung des Auges (4) entsprechende Mitverkippung der Schnittfläche (C) bestimmt ist, eine transformierte zweite Bearbeitungsbahn (t') zu bestimmen, die Bearbeitungspunkte auf der verkippten Schnittfläche (C') bestimmt, und das Scannersystem (4) derart zu steuern, dass der gepulste Laserstrahl (L) auf Bearbeitungspunkte auf der transformierten zweiten Bearbeitungsbahn (t') gelenkt wird.

2. Ophthalmologische Vorrichtung (1) nach Anspruch 1, **dadurch gekennzeichnet, dass** die Fokussieroptik (51) eingerichtet ist, eine Bearbeitungshöhe der Bearbeitungspunkte in Richtung der Projektionsachse (p) mit einer Fokusverstellgeschwindigkeit zu verstellen, dass das Scannersystem (4) eingerichtet ist, Bearbeitungspunkte mit einer bezüglich der Fokusverstellgeschwindigkeit höheren Scanngeschwindigkeit auf der Bearbeitungsbahn (t) zu verschieben, und dass der Schaltkreis (2) eingerichtet ist, die transformierte zweite Bearbeitungsbahn (t') mit Höhenänderungen in Richtung der Projektionsachse (p) zu bestimmen, welche bei einer Bewegung von Bearbeitungspunkten mit der Scanngeschwindigkeit ohne Überschreitung der Fokusverstellgeschwindigkeit durch die Fokussieroptik (51) verstellbar sind.

3. Ophthalmologische Vorrichtung (1) nach einem der Ansprüche 1 oder 2, **dadurch gekennzeichnet, dass** die erste Bearbeitungsbahn (t) in Richtung der Projektionsachse (p) eine stetige Höhenänderungskomponente aufweist, und dass der Schaltkreis (2) eingerichtet ist, die transformierte zweite Bearbeitungsbahn (t') mit einer in Richtung der Projektionsachse (p) stetig zunehmenden oder stetig abnehmenden Bearbeitungshöhenkomponente zu bestimmen.

4. Ophthalmologische Vorrichtung (1) nach einem der Ansprüche 1 bis 3, **dadurch gekennzeichnet**, die erste Bearbeitungsbahn (t) in Richtung der Projektionsachse (p) eine stetige Höhenänderungskomponente aufweist, dass der Schaltkreis (2) eingerichtet ist, Teilabschnitte der transformierten zweiten Bearbeitungsbahn (t') mit einer in Richtung der Projektionsachse (p) jeweils konstanten Bahnabschnittsbearbeitungshöhe zu bestimmen, und eine Erzeugung des gepulsten Laserstrahls (L) durch die Laserquelle (3) zu unterbinden, während die Fokussieroptik (51) die Bahnabschnittsbearbeitungshöhe zweier in Richtung der Projektionsachse (p) benachbarter Teilabschnitte der transformierten zweiten Bearbeitungsbahn (t') verstellt.

5. Ophthalmologische Vorrichtung (1) nach einem der Ansprüche 1 bis 4, **dadurch gekennzeichnet, dass** Teilabschnitte der ersten Bearbeitungsbahn in Richtung der Projektionsachse (p) jeweils eine konstante Bahnabschnittsbearbeitungshöhe aufweisen, und dass der Schaltkreis (2) eingerichtet ist, die transformierte zweite Bearbeitungsbahn (t') mit einer in Richtung der Projektionsachse (p) stetig zunehmenden oder stetig abnehmenden Bearbeitungshöhenkomponente zu bestimmen.

6. Ophthalmologische Vorrichtung (1) nach einem der Ansprüche 1 bis 5, **dadurch gekennzeichnet, dass** Teilabschnitte der ersten Bearbeitungsbahn (t) in Richtung der Projektionsachse (p) jeweils eine konstante Bahnabschnittsbearbeitungshöhe aufweisen, und dass der Schaltkreis (2) eingerichtet ist, Teilabschnitte der transformierten zweiten Bearbeitungsbahn (t') mit einer in Richtung der Projektionsachse (p) jeweils konstanten Bahnabschnittsbearbeitungshöhe zu bestimmen, und eine Erzeugung des gepulsten Laserstrahls (L) durch die Laserquelle (3) zu unterbinden, während die Fokussieroptik (51) die Bahnabschnittsbearbeitungshöhe zweier in Richtung der Projektionsachse (p) benachbarter Teilabschnitte der transformierten zweiten Bearbeitungsbahn (t') verstellt.

7. Ophthalmologische Vorrichtung (1) nach einem der Ansprüche 1 bis 3 oder 5, **dadurch gekennzeichnet, dass** der Schaltkreis (2) eingerichtet ist, die transformierte zweite Bearbeitungsbahn (t') mit einer sich in Richtung der Projektionsachse (p) abwechselnd zunehmenden und abnehmenden Bearbeitungshöhenkomponente zu bestimmen.

8. Ophthalmologische Vorrichtung (1) nach einem der Ansprüche 1 bis 7, **dadurch gekennzeichnet, dass** die erste Bearbeitungsbahn (t) gekrümmt ist und Bahnabschnitte mit mindestens einer der folgenden Formen umfasst: einen kreisrunden Bahnabschnitt, einen elliptischen Bahnabschnitt, einen parabolischen Bahnabschnitt, einen hyperbolischen Bahnabschnitt, einen spiralförmigen Bahnabschnitt, oder einen Spline-Bahnabschnitt.

9. Ophthalmologische Vorrichtung (1) nach einem der Ansprüche 1 bis 8, **dadurch gekennzeichnet, dass** der Schaltkreis (2) eingerichtet ist, die transformierte zweite Bearbeitungsbahn (t') ausgehend von der ersten Bearbeitungsbahn (t) zu bestimmen, durch Durchführung mindestens eine der folgenden Operationen: Strecken von ersten Bereichen der ersten Bearbeitungsbahn (t), Stauchen von zweiten Bereichen der ersten Bearbeitungsbahn (t), oder Unterbrechen von dritten Bereichen der ersten Bearbeitungsbahn (t).

10. Ophthalmologische Vorrichtung (1) nach einem der Ansprüche 1 bis 9, **dadurch gekennzeichnet, dass** der Schaltkreis (2) eingerichtet ist, das Scannersystem (4) zum Schneiden eines durch zwei Schnittflächen gebildeten zur Zentrumsachse (m) des Patienteninterfaces (52) symmetrischen Lentikels im Augengewebe (60) zu steuern, und bei der Verkippung des Auges (6) zur Zentrumsachse (m) des Patienteninterfaces (52), einen Scheitelpunkt (S') eines verkippten Lentikels zu ermitteln, welches durch eine der Verkippung des Auges (6) entsprechende Mitverkippung des Lentikels bestimmt ist, transformierte dritte Bearbeitungsbahnen (t') zu bestimmen, die Bearbeitungspunkte auf den Schnittflächen (C') des verkippten Lentikels bestimmen, und das Scannersystem (4) derart zu steuern, dass der gepulste Laserstrahl (L) auf Bearbeitungspunkte auf den transformierten dritten Bearbeitungsbahnen (t') gelenkt wird.

11. Ophthalmologische Vorrichtung (1) nach einem der Ansprüche 1 bis 10, **dadurch gekennzeichnet, dass** das Patienteninterface (52) einen zur Zentrumsachse (m) symmetrischen, gewölbten Innenraum (55) zur Aufnahme eines Hornhautbereichs (60) des Auges (6) aufweist.

12. Ophthalmologische Vorrichtung (1) nach einem der Ansprüche 1 bis 10, **gekennzeichnet dadurch, dass** das Patienteninterface (52) einen zur Zentrumsachse (m) symmetrischen, planaren Innenraum zur Aufnahme eines Hornhautbereichs (60) des Auges (6) aufweist.

13. Ophthalmologische Vorrichtung (1) nach einem der Ansprüche 1 bis 12, **dadurch gekennzeichnet, dass** die ophthalmologische Vorrichtung eine Messvorrichtung (7) umfasst, die eingerichtet ist, Referenzstrukturen und/oder Referenzmarkierungen im oder auf dem Augengewebe (60) zu erfassen, und dass der Schaltkreis (2) eingerichtet ist, die Verkippung des Auges (6) zur Zentrumsachse (m) des Patienteninterfaces (52) aufgrund der durch die Messvorrichtung (7) erfassten Referenzstrukturen und/oder Referenzmarkierungen zu bestimmen.

14. Ophthalmologische Vorrichtung (1) nach einem der Ansprüche 1 bis 13, **gekennzeichnet durch** eines oder mehrere Ansaugelemente (54), welche eingerichtet sind, das Patienteninterface (52) am Auge (6) zu fixieren.

15. Ophthalmologische Vorrichtung (1) nach einem der Ansprüche 1 bis 14, **dadurch gekennzeichnet, dass** das Scannersystem (4) eine erste Scannvorrichtung (42) umfasst, die eingerichtet ist, den gepulsten Laserstrahl (L) im Augengewebe (60) mit einer Vorschubgeschwindigkeit entlang einer Vorschublinie auf der Bearbeitungsbahn (t, t') zu lenken, und dass das Scannersystem (4) eine zweite Scannvorrichtung (41) umfasst, die eingerichtet ist, den gepulsten Laserstrahl (L) im Augengewebe (60) mit einer bezüglich der Vorschubgeschwindigkeit höheren Abtastgeschwindigkeit entlang einer quer zur Vorschublinie verlaufenden Abtastlinie auf der Bearbeitungsbahn (t, t') zu lenken.

## Claims

1. Ophthalmologic device (1) for processing ocular tissue (60) of an eye (6), comprising a laser source (3) configured to generate a pulsed laser beam (L); an application head (5) with focusing optics (51) and a patient interface (52), wherein the focusing optics (51) has a projection axis (p) and is configured to focus the pulsed laser beam (L) in the ocular tissue (60) onto a processing point, and wherein the patient interface (52) has a center axis (m) and is configured to fix the application head (5) to the eye; a scanner system (4) configured to direct the pulsed laser beam (L) in the ocular tissue (60) to processing points on a processing path (t); and a circuit (2) configured to control the scanner system (4) to cut a cut surface (C) in the ocular tissue (60) symmetrical to the center axis (m) of the patient interface (52), wherein the pulsed laser beam (L) is directed to processing points on the cut surface (C) on a first processing path (t),
**characterized in that** the circuit (2) is further configured to determine a vertex (S') of a tilted cut surface (C') when the eye (6) is tilted relative to the centre axis (m) of the patient interface (52), which vertex is determined by a co-tilt of the cut surface (C) corresponding to the tilting of the eye (4), to determine a transformed second processing path (t'), which determines processing points on the tilted cut surface (C'), and to control the scanner system (4) in such a way that the pulsed laser beam (L) is directed onto processing points on the transformed second processing path (t').

2. Ophthalmological device (1) according to claim 1, **characterized in that** the focusing optics (51) is configured to adjust a processing height of the processing points in the direction of the projection axis (p) at a focus adjustment speed, **in that** the scanner system (4) is configured to move processing points along the processing path (t) at a higher scanning speed with respect to the focus adjustment speed, and **in that** the circuit (2) is configured to determine the transformed second processing path (t') with height changes in the direction of the projection axis (p) which can be adjusted by the focusing optics (51) when processing points are moved at the scanning speed without exceeding the focus adjustment speed.

3. Ophthalmological device (1) according to one of claims 1 or 2, **characterized in that** the first processing path (t) has a continuous height change component in the direction of the projection axis (p), and **in that** the circuit (2) is configured to determine the transformed second processing path (t') with a continuously increasing or continuously decreasing processing height component in the direction of the projection axis (p).

4. Ophthalmological device (1) according to one of claims 1 to 3, **characterized in that** the first processing path (t) has a continuous height change component in the direction of the projection axis (p), **in that** the circuit (2) is configured to determine partial sections of the transformed second processing path (t') with a path section processing height which is continuous in each case in the direction of the projection axis (p), and to prevent generation of the pulsed laser beam (L) by the laser source (3) while the focusing optics (51) adjusts the path section processing height of two partial sections of the transformed second processing path (t') which are adjacent in the direction of the projection axis (p).

5. Ophthalmological device (1) according to one of claims 1 to 4, **characterized in that** partial sections of the first processing path each have a constant path section processing height in the direction of the projection axis (p), and **in that** the circuit (2) is configured to determine the transformed second processing path (t') with a processing height component which is continuously increasing or continuously decreasing in the direction of the projection axis (p).

6. Ophthalmological device (1) according to one of claims 1 to 5, **characterized in that** partial sections of the first processing path (t) each have a constant path section processing height in the direction of the projection axis (p), and **in that** the circuit (2) is configured to determine partial sections of the transformed second processing path (t') each having a constant path section processing height in the direction of the projection axis (p), and to prevent generation of the pulsed laser beam (L) by the laser source (3) while the focusing optics (51) adjusts the path section processing height of two partial sections of the transformed second processing path (t') which are adjacent in the direction of the projection axis (p).

7. Ophthalmological device (1) according to one of claims 1 to 3 or 5, **characterized in that** the circuit (2) is configured to determine the transformed second processing path (t') with an alternately increasing and decreasing processing height component in the direction of the projection axis (p).

8. Ophthalmological device (1) according to any one of claims 1 to 7, **characterized in that** the first processing path (t) is curved and comprises path sections having at least one of the following shapes: a circular path section, an elliptical path section, a parabolic path section, a hyperbolic path section, a spiral path section, or a spline path section.

9. The ophthalmic device (1) according to any one of claims 1 to 8, **characterized in that** the circuit (2) is configured to determine the transformed second processing path (t') starting from the first processing path (t) by performing at least one of the following operations: stretching first regions of the first processing path (t), compressing second regions of the first processing path (t), or interrupting third regions of the first processing path (t).

10. Ophthalmological device (1) according to one of claims 1 to 9, **characterized in that** the circuit (2) is configured to control the scanner system (4) for cutting a lenticule in the ocular tissue (60) which is formed by two cut surfaces symmetrical to the centre axis (m) of the patient interface (52), and to determine a vertex (S') of a tilted lenticule when the eye (6) is tilted with respect to the centre axis (m) of the patient interface (52), which is determined by a co-tilt of the lenticule corresponding to the tilting of the eye (6), to determine transformed third processing paths (t') which determine processing points on the cut surfaces (C') of the tilted lenticule, and to control the scanner system (4) in such a way that the pulsed laser beam (L) is directed onto processing points on the transformed third processing paths (t').

11. Ophthalmological device (1) according to any one of claims 1 to 10, **characterized in that** the patient interface (52) comprises a domed interior space (55) symmetrical to the centre axis (m) for receiving a corneal region (60) of the eye (6).

12. Ophthalmological device (1) according to any one of claims 1 to 10, **characterized in that** the patient interface (52) comprises a planar interior space symmetrical to the centre axis (m) for receiving a corneal region (60) of the eye (6).

13. Ophthalmological device (1) according to one of claims 1 to 12, **characterized in that** the ophthalmological device comprises a measuring device (7) configured to detect reference structures and/or reference markings in or on the ocular tissue (60), and **in that** the circuit (2) is configured to determine the tilt of the eye (6) with respect to the centre axis (m) of the patient interface (52) on the basis of the reference structures and/or reference markings detected by the measuring device (7).

14. Ophthalmological device (1) according to any one of claims 1 to 13, **characterized by** one or more suction elements (54) configured to fix the patient interface (52) to the eye (6).

15. Ophthalmological device (1) according to any one of claims 1 to 14, **characterized in that** the scanner system (4) comprises a first scanning device (42) configured to direct the pulsed laser beam (L) in the ocular tissue (60) at a feed rate along a feed line on the processing path (t, t'), and **in that** the scanner system (4) comprises a second scanning device (41) which is configured to direct the pulsed laser beam (L) in the ocular tissue (60) at a higher scanning rate with respect to the feed rate along a scanning line transversely to the feed line on the processing path (t, t').

## Revendications

1. Dispositif ophtalmologique (1) pour le traitement du tissu oculaire (60) d'un œil (6), comprenant une source laser (3) conçue pour générer un faisceau laser pulsé (L) ; une tête d'application (5) avec une optique de focalisation (51) et une interface patient (52), l'optique de focalisation (51) présentant un axe de projection (p) et étant conçue pour focaliser le faisceau laser pulsé (L) dans le tissu oculaire (60) sur un point de traitement, et l'interface patient (52) présentant un axe de centrage (m) et étant conçue pour fixer la tête d'application (5) sur l'œil ; un système de balayage (4) conçu pour diriger le faisceau laser pulsé (L) dans le tissu oculaire (60) sur des points de traitement sur une trajectoire de traitement (t) ; et un circuit (2) conçu pour commander le système de balayage (4) pour découper une surface de coupe (C) symétrique à l'axe de centrage (m) de l'interface patient (52) dans le tissu oculaire (60), le faisceau laser pulsé (L) étant dirigé sur des points de traitement sur la surface de coupe (C) sur une première trajectoire de traitement (t),
**caractérisé en ce que** le circuit (2) est en outre conçu pour déterminer, en cas de basculement de l'oeil (6) par rapport à l'axe de centrage (m) de l'interface patient (52), un point sommital (S') d'une surface de coupe basculée (C'), qui est déterminé par un co-basculement de la surface de coupe (C) correspondant au basculement de l'oeil (4), déterminer une deuxième trajectoire de traitement (t') transformée, qui détermine des points de traitement sur la surface de coupe basculée (C'), et commander le système de balayage (4) de telle sorte que le faisceau laser pulsé (L) soit dirigé sur des points de traitement sur la deuxième trajectoire de traitement transformée (t').

2. Dispositif ophtalmologique (1) selon la revendication 1, **caractérisé en ce que** l'optique de focalisation (51) est conçue pour régler une hauteur de traitement des points de traitement dans la direction de l'axe de projection (p) avec une vitesse de balayage de focalisation, **en ce que** le système de balayage (4) est conçu pour déplacer les points de traitement sur la trajectoire de traitement (t) avec une vitesse de balayage plus élevée par rapport à la vitesse de balayage de focalisation, et **en ce que** le circuit (2) est conçu pour déterminer la deuxième trajectoire de traitement (t') transformée avec des modifications de hauteur dans la direction de l'axe de projection (p), qui sont réglables par l'optique de focalisation (51) lors d'un déplacement de points de traitement à la vitesse de balayage sans dépassement de la vitesse de balayage de focalisation.

3. Dispositif ophtalmologique (1) selon l'une des revendications 1 ou 2, **caractérisé en ce que** la première trajectoire de traitement (t) présente une composante de variation de hauteur continue dans la direction de l'axe de projection (p), et **en ce que** le circuit (2) est conçu pour déterminer la deuxième trajectoire de traitement (t') transformée avec une composante de variation de hauteur de traitement qui augmente de manière continue ou qui diminue de manière continue dans la direction de l'axe de projection (p).

4. Dispositif ophtalmologique (1) selon l'une des revendications 1 à 3, **caractérisé en ce que** la première trajectoire de traitement (t) présente une composante de variation de hauteur continue dans la direction de l'axe de projection (p), **en ce que** le circuit (2) est conçu pour déterminer des sections partielles de la deuxième trajectoire de traitement (t') transformée avec une hauteur de traitement de section de trajectoire respectivement constante dans la direction de l'axe de projection (p), et d'empêcher une génération du faisceau laser pulsé (L) par la source laser (3), tandis que l'optique de focalisation (51) règle la hauteur de traitement de section de trajectoire de deux sections partielles de la deuxième trajectoire de traitement transformée (t') voisines dans la direction de l'axe de projection (p).

5. Dispositif ophtalmologique (1) selon l'une des revendications 1 à 4, **caractérisé en ce que** des sections partielles de la première trajectoire de traitement présentent chacune une hauteur de traitement de section de trajectoire constante dans la direction de l'axe de projection (p), et **en ce que** le circuit (2) est conçu pour déterminer la deuxième trajectoire de traitement transformée (t') avec une composante de hauteur de traitement augmentant continuellement ou diminuant continuellement dans la direction de l'axe de projection (p).

6. Dispositif ophtalmologique (1) selon l'une des revendications 1 à 5, **caractérisé en ce que** des sections partielles de la première trajectoire de traitement (t) présentent chacune une hauteur de traitement de section de trajectoire constante dans la direction de l'axe de projection (p), et **en ce que** le circuit (2) est conçu pour déterminer des sections partielles de la deuxième trajectoire de traitement (t') transformée présentant chacune une hauteur de traitement de section de trajectoire constante dans la direction de l'axe de projection (p), et d'empêcher une génération du faisceau laser pulsé (L) par la source laser (3), tandis que l'optique de focalisation (51) règle la hauteur de traitement de section de trajectoire de deux sections partielles de la deuxième trajectoire de traitement transformée (t') voisines dans la direction de l'axe de projection (p).

7. Dispositif ophtalmologique (1) selon l'une des revendications 1 à 3 ou 5, **caractérisé en ce que** le circuit (2) est conçu pour déterminer la deuxième trajectoire de traitement (t') transformée avec une composante de hauteur de traitement qui augmente et diminue alternativement dans la direction de l'axe de projection (p).

8. Dispositif ophtalmologique (1) selon l'une quelconque des revendications 1 à 7, **caractérisé en ce que** la première trajectoire de traitement (t) est courbe et comprend des portions de trajectoire ayant au moins l'une des formes suivantes : une portion de trajectoire circulaire, une portion de trajectoire elliptique, une portion de trajectoire parabolique, une portion de trajectoire hyperbolique, une portion de trajectoire spiroïdale, ou une portion de trajectoire spline.

9. Dispositif ophtalmologique (1) selon l'une des revendications 1 à 8, **caractérisé en ce que** le circuit (2) est conçu pour déterminer la deuxième trajectoire de traitement (t') transformée à partir de la première trajectoire de traitement (t), en effectuant au moins l'une des opérations suivantes : l'étirement de premières zones de la première trajectoire de traitement (t), le refoulement de deuxièmes zones de la première trajectoire de traitement (t), ou l'interruption de troisièmes zones de la première trajectoire de traitement (t).

10. Dispositif ophtalmologique (1) selon l'une des revendications 1 à 9, **caractérisé en ce que** le circuit (2) est conçu pour commander le système de balayage (4) pour découper dans le tissu oculaire (60) un lenticule formé par deux surfaces de coupe symétriques par rapport à l'axe de centrage (m) de l'interface patient (52), et pour déterminer un point sommital (S') d'un lenticule basculé lors du basculement de l'oeil (6) par rapport à l'axe de centrage (m) de l'interface patient (52), qui est déterminé par un co-basculement de l'élément lenticulaire correspondant au basculement de l'oeil (6), à déterminer des troisièmes trajectoires de traitement transformées (t') qui déterminent des points de traitement sur les surfaces de traitement (C') de l'élément lenticulaire basculé, et à commander le système de balayage (4) de telle sorte que le faisceau laser pulsé (L) soit dirigé sur des points de traitement sur les troisièmes trajectoires de traitement transformées (t').

11. Dispositif ophtalmologique (1) selon l'une des revendications 1 à 10, **caractérisé en ce que** l'interface patient (52) présente un espace intérieur (55) bombé, symétrique par rapport à l'axe de centrage (m), destiné à recevoir une zone cornéenne (60) de l'œil (6).

12. Dispositif ophtalmologique (1) selon l'une des revendications 1 à 10, **caractérisé en ce que** l'interface patient (52) présente un espace intérieur plan, symétrique par rapport à l'axe de centrage (m), destiné à recevoir une zone cornéenne (60) de l'œil (6).

13. Dispositif ophtalmologique (1) selon l'une des revendications 1 à 12, **caractérisé en ce que** le dispositif ophtalmologique comprend un dispositif de mesure (7) qui est conçu pour détecter des structures de référence et/ou des marquages de référence dans ou sur le tissu oculaire (60), et **en ce que** le circuit (2) est conçu pour déterminer le basculement de l'œil (6) par rapport à l'axe de centrage (m) de l'interface patient (52) sur la base des structures de référence et/ou des marquages de référence détectés par le dispositif de mesure (7).

14. Dispositif ophtalmologique (1) selon l'une des revendications 1 à 13, **caractérisé par** un ou plusieurs éléments d'aspiration (54) qui sont conçus pour fixer l'interface patient (52) sur l'œil (6).

15. Dispositif ophtalmologique (1) selon l'une des revendications 1 à 14, **caractérisé en ce que** le système de balayage (4) comprend un premier dispositif de balayage (42) qui est conçu pour diriger le faisceau laser pulsé (L) dans le tissu oculaire (60) à une vitesse d'avance le long d'une ligne d'avance sur la trajectoire de traitement (t, t'), et **en ce que** le système de balayage (4) comprend un deuxième dispositif de balayage (41) qui est conçu pour diriger le faisceau laser pulsé (L) dans le tissu oculaire (60) avec une vitesse de balayage plus élevée par rapport à la vitesse d'avance le long d'une ligne de balayage s'étendant transversalement à la ligne d'avance sur la trajectoire de traitement (t, t').
